# EUROPEAN PATENT APPLICATION

(11) **EP 3 643 725 A1**
(43) Date of publication of application: **29.04.2020**
(21) Application number: 18821563.6
(22) Date of filing: 20.06.2018
(51) Int. Cl.: C07K 16/18, A61K 39/395, A61P 35/00, G01N 33/574, C12N 15/13

(54) **ANTISULFATAED GLYCOSAMINOGLYCAN ANTIBODY**

(30) Priority: 20.06.2017 JP 2017120435
(71) Applicant: Savid Therapeutics Inc., Tokyo 168-0064 (JP)
(72) Inventor: ISHIKAWA, Shumpei, Tokyo 113-8510 (JP); KATOH, Hiroto, Tokyo 113-8510 (JP); KOMURA, Daisuke, Tokyo 113-8510 (JP)
(74) Representative: CH Kilger Anwaltspartnerschaft mbB
(86) International application number: PCT/JP2018/023425
(87) International publication number: WO 2018/235855

(57) **Abstract**

To provide a novel immunotherapeutic agent which can be used for treatment of gastric cancer and the like including diffuse-type gastric carcinoma. An antibody that binds to sulfated glycosaminoglycan and has cell growth inhibitory activity is provided. The antibody of the present invention can be used as a cell growth inhibitor, a targeting reagent for drug delivery, or an agent for detecting cancer.

SEQUENCE LISTING

<110>  National University Corporation Tokyo Medical and Dental
       University

<120>  Anti-Sulfated Glycosaminoglycan Antibody

<130>  PH-7491-PCT

<150>  JP 2017-120435
<151>  2017-06-20

<160>  31

<170>  PatentIn version 3.5

<210>  1
<211>  118
<212>  PRT
<213>  Artificial

<220>
<223>  Gpat#1 Antibody Heavy Chain Variable Region

<400>  1

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser
        35                  40                  45

Ser Trp Asp Ser Gly Ser Ile Ala Tyr Ala Asp Ser Val Lys Gly Arg
    50                  55                  60

Phe Ile Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Pro Tyr Gly Met Asp Val Trp Gly Gln Gly Thr
            100                 105                 110

Thr Val Thr Val Ser Ser
        115


<210>  2
<211>  113
<212>  PRT
<213>  Artificial

<220>
<223>  Gpat#1 Antibody Light Chain Variable Region

<400>  2

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Asn Ile Phe Tyr Ser
            20                  25                  30

Ser Asn Asn Asp Asn Tyr Leu Ala Trp Tyr Gln Gln Lys Pro Gly Gln
        35                  40                  45

Pro Pro Lys Leu Leu Phe Tyr Arg Ala Ser Thr Trp Glu Ser Gly Val
    50                  55                  60

Pro Asp Arg Phe Ser Ala Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Tyr Thr Phe Gly Gln Gly Thr Lys Leu Glu Ile
            100                 105                 110

Lys

<210>  3
<211>  309
<212>  DNA
<213>  Homo sapiens

<400>  3
agactctcct gtgcagcctc tggattcagc ttggatgatt atgccatgca ctgggtccgg      60

caagctccag ggaagggcct ggagtgggtc tcaggtagta gttgggatag tggtagcata     120

gcctatgcgg actctgtgaa gggccgattc atcatctcca gagacaacgc caagaactcc     180

ctgtatctgc aaatgaacag tctgagagct gaggacacgg ccttgtatta ctgtgcaaaa     240

ggccttctgc cctccttgcc ctatggaatg gacgtctggg gccaagggac cacggtcacc     300

gtctcctca                                                             309

<210>  4
<211>  103
<212>  PRT
<213>  Homo sapiens

<400>  4

Arg Leu Ser Cys Ala Ala Ser Gly Phe Ser Leu Asp Asp Tyr Ala Met
1               5                   10                  15

His Trp Val Arg Gln Ala Pro Gly Lys Gly Leu Glu Trp Val Ser Gly
            20                  25                  30

Ser Ser Trp Asp Ser Gly Ser Ile Ala Tyr Ala Asp Ser Val Lys Gly
        35                  40                  45

Arg Phe Ile Ile Ser Arg Asp Asn Ala Lys Asn Ser Leu Tyr Leu Gln
    50                  55                  60

Met Asn Ser Leu Arg Ala Glu Asp Thr Ala Leu Tyr Tyr Cys Ala Lys
65                  70                  75                  80

Gly Leu Leu Pro Ser Leu Pro Tyr Gly Met Asp Val Trp Gly Gln Gly
                85                  90                  95

Thr Thr Val Thr Val Ser Ser
            100

<210>  5
<211>  288
<212>  DNA
<213>  Homo sapiens

<400>  5
agggccacca tcaactgcaa gtccagccag aatattttct acagctccaa caatgacaat      60

tacttagctt ggtaccagca gaaaccggga cagcctccta agttgctctt ttacagggca     120

tctacctggg aatccggggt ccctgaccga ttcagtgcca gcgggtctgg gacagatttc     180

actctcacca tcagcagcct gcaggctgaa gatgtggcag tttattattg tcagcaatat     240

tatagtactc cgtacacttt tggccagggg accaagctgg agatcaaa                  288

<210>  6
<211>  96
<212>  PRT
<213>  Homo sapiens

<400>  6

Arg Ala Thr Ile Asn Cys Lys Ser Ser Gln Asn Ile Phe Tyr Ser Ser
1               5                   10                  15

Asn Asn Asp Asn Tyr Leu Ala Trp Tyr Gln Gln Lys Pro Gly Gln Pro
            20                  25                  30

Pro Lys Leu Leu Phe Tyr Arg Ala Ser Thr Trp Glu Ser Gly Val Pro
        35                  40                  45

Asp Arg Phe Ser Ala Ser Gly Ser Gly Thr Asp Phe Thr Leu Thr Ile
    50                  55                  60

Ser Ser Leu Gln Ala Glu Asp Val Ala Val Tyr Tyr Cys Gln Gln Tyr
65                  70                  75                  80

Tyr Ser Thr Pro Tyr Thr Phe Gly Gln Gly Thr Lys Leu Glu Ile Lys
                85                  90                  95

<210>  7
<211>  124
<212>  PRT
<213>  Artificial

<220>
<223>  Gpat#2 Antibody Heavy Chain Variable Region

<400>  7

Gln Val Gln Leu Gln Glu Ser Gly Pro Gly Leu Val Lys Pro Ser Glu
1               5                   10                  15

Thr Leu Ser Leu Thr Cys Thr Val Ser Gly Ala Ser Ile Arg Ser Thr
            20                  25                  30

Ala Phe Tyr Trp Gly Trp Ile Arg Gln Ser Ala Ala Lys Gly Leu Glu
        35                  40                  45

Trp Ile Gly His Val Tyr Gln Ser Gly Thr Ser Lys Tyr Asn Pro Ser
    50                  55                  60

Leu Asn Ser Arg Val Thr Ile Ser Leu Asp Thr Ser Lys Asn Gln Phe
65                  70                  75                  80

Phe Leu Arg Leu Thr Ser Val Thr Ala Ala Asp Thr Ala Ile Tyr Tyr
                85                  90                  95

Cys Ala Ser Ser Gln Ala Tyr Tyr Asp Val Ser Gly Gly Pro Val Asn
            100                 105                 110

Asn Trp Gly Arg Gly Thr Leu Val Ala Val Ser Ser
        115                 120

<210>  8
<211>  111
<212>  PRT
<213>  Artificial

<220>
<223>  Gpat#2 Antibody Light Chain Variable Region

<400>  8

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val Tyr Thr
            20                  25                  30

Asp Gly Asn Thr Tyr Leu Asn Trp Tyr His Gln Arg Pro Gly Gln Ser
        35                  40                  45

Pro Arg Arg Leu Ile Tyr Lys Val Ser Asn Arg Asp Ser Gly Val Pro
    50                  55                  60

Asp Arg Phe Ser Gly Ser Gly Ser Gly Thr Asp Phe Thr Leu Lys Ile
65                  70                  75                  80

Thr Arg Val Glu Ala Asp Asp Ile Gly Leu Tyr Tyr Cys Ile Gln Gly
                85                  90                  95

Thr His Trp Pro Pro Phe Gly Gln Gly Thr Lys Leu Glu Ile Arg
            100                 105                 110

<210>  9
<211>  324
<212>  DNA
<213>  Homo sapiens

<400>  9
accctgtccc tcacctgcac tgtctctggt gcctccatca ggagtactgc tttttactgg      60

gggtggatcc gccagtcagc agcgaagggg ctggagtgga ttggccatgt ctatcaaagt     120

ggaacttcca aatacaaccc gtccctcaac agtcgagtca ccatatcgct ggacacatcc     180

aagaaccagt tcttcctcag actaacctct gtgaccgccg cggacacggc catatattat     240

tgtgcgagta gtcaggcgta ttacgatgtt tcgggcggcc ccgttaataa ctggggccgg     300

ggaaccctgg tcgccgtctc ctca                                            324

<210>  10
<211>  108
<212>  PRT
<213>  Homo sapiens

<400>  10

Thr Leu Ser Leu Thr Cys Thr Val Ser Gly Ala Ser Ile Arg Ser Thr
1               5                   10                  15

Ala Phe Tyr Trp Gly Trp Ile Arg Gln Ser Ala Ala Lys Gly Leu Glu
            20                  25                  30

Trp Ile Gly His Val Tyr Gln Ser Gly Thr Ser Lys Tyr Asn Pro Ser
        35                  40                  45

Leu Asn Ser Arg Val Thr Ile Ser Leu Asp Thr Ser Lys Asn Gln Phe
    50                  55                  60

Phe Leu Arg Leu Thr Ser Val Thr Ala Ala Asp Thr Ala Ile Tyr Tyr
65                  70                  75                  80

Cys Ala Ser Ser Gln Ala Tyr Tyr Asp Val Ser Gly Gly Pro Val Asn
                85                  90                  95

Asn Trp Gly Arg Gly Thr Leu Val Ala Val Ser Ser
            100                 105

<210> 11
<211> 282
<212> DNA
<213> Homo sapiens

<400> 11
ccggcctcca tctcctgcag gtctagtcaa agcctcgttt acactgatgg aaacacctac 60

ttgaattggt atcaccagag gccaggccag tctccaaggc gcctaattta taaggtttct 120

aaccgggact ctggggtccc agacagattc agcggcagtg ggtcaggcac tgatttcaca 180

ctgaaaatca ccagggtgga ggctgacgat attggacttt attactgcat ccaaggtaca 240

cactggcctc cgttcggcca agggaccaag ttggaaatca ga 282

<210> 12
<211> 94
<212> PRT
<213> Homo sapiens

<400> 12

Pro Ala Ser Ile Ser Cys Arg Ser Ser Gln Ser Leu Val Tyr Thr Asp
1               5                   10                  15

Gly Asn Thr Tyr Leu Asn Trp Tyr His Gln Arg Pro Gly Gln Ser Pro
            20                  25                  30

Arg Arg Leu Ile Tyr Lys Val Ser Asn Arg Asp Ser Gly Val Pro Asp
        35                  40                  45

Arg Phe Ser Gly Ser Gly Ser Gly Thr Asp Phe Thr Leu Lys Ile Thr
    50                  55                  60

Arg Val Glu Ala Asp Asp Ile Gly Leu Tyr Tyr Cys Ile Gln Gly Thr
65                  70                  75                  80

His Trp Pro Pro Phe Gly Gln Gly Thr Lys Leu Glu Ile Arg
                85                  90

<210>  13
<211>  8
<212>  PRT
<213>  Homo sapiens

<400>  13

Gly Phe Ser Leu Asp Asp Tyr Ala
1               5

<210>  14
<211>  8
<212>  PRT
<213>  Homo sapiens

<400>  14

Ser Ser Trp Asp Ser Gly Ser Ile
1               5

<210>  15
<211>  14
<212>  PRT
<213>  Homo sapiens

<400>  15

Ala Lys Gly Leu Leu Pro Ser Leu Pro Tyr Gly Met Asp Val
1               5                   10

<210>  16
<211>  12
<212>  PRT
<213>  Homo sapiens

<400>  16

Gln Asn Ile Phe Tyr Ser Ser Asn Asn Asp Asn Tyr
1               5                   10

<210>  17
<211>  3
<212>  PRT
<213>  Homo sapiens

<400>  17

Arg Ala Ser
1

<210>  18
<211>  9

<212>  PRT
<213>  Homo sapiens

<400>  18

Gln Gln Tyr Tyr Ser Thr Pro Tyr Thr
1               5


<210>  19
<211>  10
<212>  PRT
<213>  Homo sapiens

<400>  19

Gly Ala Ser Ile Arg Ser Thr Ala Phe Tyr
1               5                   10


<210>  20
<211>  7
<212>  PRT
<213>  Homo sapiens

<400>  20

Val Tyr Gln Ser Gly Thr Ser
1               5


<210>  21
<211>  16
<212>  PRT
<213>  Homo sapiens

<400>  21

Ala Ser Ser Gln Ala Tyr Tyr Asp Val Ser Gly Gly Pro Val Asn Asn
1               5                   10                  15


<210>  22
<211>  11
<212>  PRT
<213>  Homo sapiens

<400>  22

Gln Ser Leu Val Tyr Thr Asp Gly Asn Thr Tyr
1               5                   10


<210>  23
<211>  3
<212>  PRT
<213>  Homo sapiens

<400>  23

Lys Val Ser
1

<210>  24
<211>  8
<212>  PRT
<213>  Homo sapiens

<400>  24

Ile Gln Gly Thr His Trp Pro Pro
1               5


<210>  25
<211>  120
<212>  PRT
<213>  Homo sapiens

<400>  25

Gln Val Gln Leu Gln Glu Ser Gly Pro Gly Leu Val Lys Pro Ser Glu
1               5                   10                  15


Thr Leu Ser Leu Thr Cys Ser Val Ser Gly Gly Ser Ile Ser Ser His
            20                  25                  30


Tyr Trp Ser Trp Ile Arg Gln Pro Pro Gly Lys Ala Leu Glu Trp Ile
        35                  40                  45


Gly His Ile Phe Tyr Ser Gly Ser Ala Thr Tyr Asn Pro Ser Leu Lys
    50                  55                  60


Ser Arg Val Thr Ile Ser Val Gly Thr Ser Lys Asn Asp Phe Thr Leu
65                  70                  75                  80


Lys Leu Arg Ser Val Thr Ala Ala Asp Thr Ala Val Tyr Tyr Cys Ala
                85                  90                  95


Arg Phe Leu Ser Asp Tyr Ser Ser Gly Arg Phe Asp Tyr Trp Gly Gln
            100                 105                 110


Gly Thr Pro Val Thr Val Ser Ser
        115                 120


<210>  26
<211>  112
<212>  PRT
<213>  Homo sapiens

<400>  26

Asp Ile Val Met Thr Gln Thr Pro Leu Ser Ser Pro Val Thr Leu Gly
1               5                   10                  15


Gln Pro Ala Ser Ile Ser Cys Arg Ser Ser Gln Ser Leu Val His Ser

20                  25                  30

Asp Gly Asn Thr Tyr Leu Gly Trp Leu Gln Lys Arg Pro Gly Gln Pro
        35                  40                  45

Pro Arg Leu Leu Ile Tyr Lys Ile Ser Asn Arg Leu Ser Gly Val Pro
    50                  55                  60

Asp Arg Phe Ser Gly Ser Gly Ala Gly Thr Asp Phe Thr Leu Lys Ile
65                  70                  75                  80

Ser Arg Val Glu Ala Glu Asp Val Gly Val Tyr Tyr Cys Leu Gln Ala
                85                  90                  95

Thr Gln Phe Pro Trp Thr Phe Gly Gln Gly Thr Lys Val Glu Ile Lys
            100                 105                 110

<210>  27
<211>  118
<212>  PRT
<213>  Artificial

<220>
<223>  Modified Antibody Heavy Chain Variable Region

<400>  27

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser
        35                  40                  45

Ser Trp Asn Ser Gly Ser Ile Ala Tyr Ala Asp Ser Val Lys Gly Arg
    50                  55                  60

Phe Ile Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Pro Tyr Gly Met Asp Val Trp Gly Gln Gly Thr
            100                 105                 110

Thr Val Thr Val Ser Ser
        115

<210>  28
<211>  118
<212>  PRT
<213>  Artificial

<220>
<223>  Modified Antibody Heavy Chain Variable Region

<400>  28

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser
        35                  40                  45

Ser Trp Asp Ser Gly Thr Ile Ala Tyr Ala Asp Ser Val Lys Gly Arg
    50                  55                  60

Phe Ile Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Pro Tyr Gly Met Asp Val Trp Gly Gln Gly Thr
            100                 105                 110

Thr Val Thr Val Ser Ser
        115


<210>  29
<211>  118
<212>  PRT
<213>  Artificial

<220>
<223>  Modified Antibody Heavy Chain Variable Region

<400>  29

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser

35                  40                  45

Ser Trp Asp Ser Gly Ser Ile Gly Tyr Ala Asp Ser Val Lys Gly Arg
    50                  55                  60

Phe Ile Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Pro Tyr Gly Met Asp Val Trp Gly Gln Gly Thr
            100                 105                 110

Thr Val Thr Val Ser Ser
        115

<210>  30
<211>  118
<212>  PRT
<213>  Artificial

<220>
<223>  Modified Antibody Heavy Chain Variable Region

<400>  30

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser
        35                  40                  45

Ser Trp Asp Ser Gly Ser Ile Ala Tyr Ala Asp Ser Val Lys Gly Arg
    50                  55                  60

Phe Thr Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Pro Tyr Gly Met Asp Val Trp Gly Gln Gly Thr
            100                 105                 110

Thr Val Thr Val Ser Ser
        115

<210>  31
<211>  118
<212>  PRT
<213>  Artificial

<220>
<223>  Modified Antibody Heavy Chain Variable Region

<400>  31

Glu Val Gln Leu Val Glu Ser Gly Gly Gly Leu Val Gln Pro Gly Arg
1               5                   10                  15

Leu Ser Cys Ala Ala Ser Gly Phe Ser Leu Asp Asp Tyr Ala Met His
            20                  25                  30

Trp Val Arg Gln Ala Pro Gly Lys Gly Leu Glu Trp Val Ser Gly Ser
        35                  40                  45

Ser Trp Asp Ser Gly Ser Ile Ala Tyr Ala Asp Ser Val Lys Gly Arg
    50                  55                  60

Phe Ile Ile Ser Arg Asp Asn Ala Lys Asn Ser Leu Tyr Leu Gln Met
65                  70                  75                  80

Asn Ser Leu Arg Ala Glu Asp Thr Ala Leu Tyr Tyr Cys Ala Lys Gly
                85                  90                  95

Leu Leu Pro Ser Leu Ser Tyr Gly Met Asp Val Trp Gly Gln Gly Thr
            100                 105                 110

Thr Val Thr Val Ser Ser
        115

## Description

### Technical Field

The present invention relates to an antibody that binds to sulfated glycosaminoglycan. The antibody of the present invention has cell growth inhibitory activity and can be used as a cell growth inhibitor, a targeting reagent for drug delivery, and an agent for detecting cancer.

### Background Art

Glycosaminoglycan is a long linear polysaccharide consisting of repeating disaccharide units, wherein the repeating unit consists of an amino sugar along with uronic acid or galactose. It has been known that glycosaminoglycan is present in various tissues in the body of animals. Glycosaminoglycan is classified based on the types of constituent sugars, the presence or absence of sulfate groups, the degree and site of sulfate groups, the binding scheme of constituent sugars, etc. As main examples of glycosaminoglycan, hyaluronic acid, chondroitin sulfate, dermatan sulfate, keratan sulfate, heparan sulfate, and heparin have been known. Heparan sulfate glycosaminoglycan is a polysaccharide consisting of a repeating disaccharide structure, to which sulfate groups bind at various levels, and typically, it is present in the form of a proteoglycan that binds to a protein called "core protein" (Non-Patent Document 1). Heparin also binds to a core protein to form a proteoglycan, and is characterized in that it has a higher degree of sulfation than heparan sulfate (Non-Patent Document 2).

Non-Patent Document 3 summarizes the correlation between the presence of glycosaminoglycan and tumor, and it describes that glycosaminoglycan can be a therapeutic target for tumor. In addition, it is disclosed that a conjugate of a malaria-derived peptide binding to chondroitin sulfate and a diphtheria toxin inhibits the growth of tumor cells *in vivo* (Non-Patent Document 4).

Gastric cancer is one of malignant tumors, which is found most frequently over the world, and among others, diffuse-type gastric carcinoma (DGC) has been known to have subtypes causing extremely poor prognosis (for example, Non-Patent Document 5). As treatments of gastric cancer, surgeries including endoscopic resection, the use of anticancer agents, and further, depending on the situation, chemotherapy involving the combined use of Trastuzumab, have been conducted.

### Prior Art Documents

### Non-Patent Documents

Non-Patent Document 1: Sasisekharan R et al., Nat Rev Cancer 2002 Jul; 2(7): 521-8
Non-Patent Document 2: Mulloy et al., Pharmacol Review 68, 76-141, January 2016
Non-Patent Document 3: Yip et al., Mol Cancer Ther 2006; 5(9) 2139-2148
Non-Patent Document 4: Salanti et al., Cancer Cell 28, 500-514, October 12, 2015
Non-Patent Document 5: Koriyama et al., World J Castroenterol 2007, 13, 3925-3931

### Summary of Invention

When a molecule that is specifically expressed in cancer is known, immunotherapy targeting the molecule can be selected as a therapeutic option. Because of its specificity, immunotherapy hardly causes harmful side effects, in comparison to chemotherapy using common anticancer agents, and thus, immunotherapy may become a promising therapeutic method.

However, it has been known that diffuse-type gastric carcinoma has a low frequency of somatic mutation. In addition, therapeutic strategy targeting mutant proteins, which has been partially reported, has not yet been effective for diffuse-type gastric carcinoma. Further, the effectiveness of immune checkpoint inhibitors, which have attracted attention in recent years, has not yet been clarified. Accordingly, it has been desired to develop a novel therapeutic method, which is effective for gastric cancer including diffuse-type gastric carcinoma and which is based on antitumor immunity.

In the aforementioned prior art documents, the correlation of glycosaminoglycan with tumor and possible cancer therapy using a peptide binding to glycosaminoglycan are disclosed. However, these prior art documents neither teach nor suggest a specific monoclonal antibody that can be used as an antibody drug.

The present inventors have conducted various studies regarding the characteristics of immune cells infiltrating into tumor in the case of human diffuse-type gastric carcinoma. As a result, the present inventors have discovered the growth of a specific B cell clone group, and could obtain the gene sequence information of the clone group that encodes antibody molecules generated. Antibody molecules have been reconstituted on the basis of the obtained information, and an antigen recognized by the antibody has been then searched. As a result, it has been surprisingly discovered that the antigen is not a protein, but is sulfated glycosaminoglycan as a polysaccharide. Moreover, the present inventors have confirmed that the antibody exhibits growth inhibitory effects on various human cell lines such as gastric cancer, colon cancer, liver cancer, pancreatic cancer, and lung cancer. The present inventors have found that the anti-sulfated glycosaminoglycan antibody of the present invention has cell growth inhibitory activity even in the absence of an effector cell and a complement and thus, the present anti-sulfated glycosaminoglycan antibody may be useful for the treatment of tumor even it is used alone, and that the present anti-sulfated glycosaminoglycan antibody is combined with other drugs such as an anticancer agent, so that it can also be used as a targeting reagent for drug delivery.

The present invention has been made based on these findings, and provides the following inventions 1 to 12.
1. An antibody that binds to sulfated glycosaminoglycan and has cell growth inhibitory activity.
2. The antibody according to the above 1, wherein the sulfated glycosaminoglycan is heparan sulfate glycosaminoglycan or heparin.
3. The antibody according to the above 1, wherein the sulfated glycosaminoglycan is heparin.
4. The antibody according to any one of the above 1 to 3, which has cell growth inhibitory activity in the absence of an effector cell and a complement.
5. An antibody binding to sulfated glycosaminoglycan, which is any one selected from the following (1) to (10):
   (1) an antibody having a heavy chain variable region comprising CDR1 consisting of the amino acid sequence set forth in SEQ ID NO: 13, CDR2 consisting of the amino acid sequence set forth in SEQ ID NO: 14, and CDR3 consisting of the amino acid sequence set forth in SEQ ID NO: 15;
   (2) an antibody having a light chain variable region comprising CDR1 consisting of the amino acid sequence set forth in SEQ ID NO: 16, CDR2 consisting of the amino acid sequence set forth in SEQ ID NO: 17, and CDR3 consisting of the amino acid sequence set forth in SEQ ID NO: 18;
   (3) an antibody having a heavy chain variable region comprising CDR1 consisting of the amino acid sequence set forth in SEQ ID NO: 19, CDR2 consisting of the amino acid sequence set forth in SEQ ID NO: 20, and CDR3 consisting of the amino acid sequence set forth in SEQ ID NO: 21;
   (4) an antibody having a light chain variable region comprising CDR1 consisting of the amino acid sequence set forth in SEQ ID NO: 22, CDR2 consisting of the amino acid sequence set forth in SEQ ID NO: 23, and CDR3 consisting of the amino acid sequence set forth in SEQ ID NO: 24;
   (5) an antibody having a heavy chain variable region having the amino acid sequence set forth in SEQ ID NO: 1 or 4, and a light chain variable region having the amino acid sequence set forth in SEQ ID NO: 2 or 6;
   (6) an antibody having a heavy chain variable region having the amino acid sequence set forth in SEQ ID NO: 7 or 10, and a light chain variable region having the amino acid sequence set forth in SEQ ID NO: 8 or 12;
   (7) an antibody having a heavy chain variable region consisting of an amino acid sequence having a sequence identity of 90% or more to SEQ ID NO: 1, and a light chain variable region consisting of an amino acid sequence having a sequence identity of 90% or more to SEQ ID NO: 2;
   (8) an antibody having a heavy chain variable region consisting of an amino acid sequence having a sequence identity of 90% or more to SEQ ID NO: 7, and a light chain variable region consisting of an amino acid sequence having a sequence identity of 90% or more to SEQ ID NO: 8;
   (9) an antibody having a heavy chain and/or a light chain consisting of an amino acid sequence comprising a substitution, deletion, and/or addition of one or several amino acids in the heavy chain variable regions and/or the light chain variable regions of the antibodies (1) to (6); and
   (10) the antibody (9) having one or more substitutions selected from the substitution of the amino acid at position 51 Asp with Asn, the substitution of the amino acid at position 56 Ala with Gly, and the substitution of the amino acid at position 66 Ile with Thr, in the amino acid sequence set forth in SEQ ID NO: 1.
6. A cell growth inhibitor comprising the antibody according to any one of the above 1 to 5 as an active ingredient.
7. The cell growth inhibitor according to the above 6, which is used in combination with a drug having cytotoxicity.
8. The cell growth inhibitor according to the above 6 or 7, which is used to inhibit the growth of cancer cells.
9. The cell growth inhibitor according to the above 8, wherein the cancer cells are cancer cells in gastric cancer, pancreatic cancer, colon cancer, liver cancer, lung cancer, and/or malignant pleural tumor.
10. A targeting reagent for drug delivery, comprising the antibody according to any one of the above 1 to 5.
11. A pharmaceutical composition comprising, as an active ingredient, the cell growth inhibitor according to any one of the above 6 to 9, or the targeting reagent according to c the above 10.
12. An agent for detecting cancer, comprising the antibody according to any one of the above 1 to 5.
13. The detecting agent according to the above 12, for use in detecting cancer selected from gastric cancer, pancreatic cancer, colon cancer, liver cancer, lung cancer, and malignant pleural tumor.
14. A method for treating cancer in a subject, comprising administering the cell growth inhibitor according to any one of the above 6 to 9, the targeting reagent according to the above 10, or the pharmaceutical composition according to the above 11, to the subject.

The present description includes the contents as disclosed in Japanese Patent Application No. 2017-120435, which is a priority document of the present application.

### Advantageous Effects of Invention

According to the present invention, provided is a novel antibody, which binds to sulfated glycosaminoglycan existing in human cancer tissues, so that it can inhibit the cell growth thereof, and can also be used for drug delivery into cells.

### Brief Description of Drawings

Fig. 1A shows a relative frequency distribution of V-J combinations of a gene encoding the heavy chain (IgG) of an antibody dominantly present in tumor tissues in a human gastric cancer case. The peak indicated with the arrow corresponds to the sequence of a Gpat#2 heavy chain gene.
Fig. 1B shows a relative frequency distribution of V-J combinations of a gene encoding the light chain (Igκ) of an antibody dominantly present in tumor tissues in a human gastric cancer case. The peak indicated with the arrow corresponds to the sequence of a Gpat#2 light chain gene.
Fig. 2A shows that a Cy5-labeled Gpat#1 antibody binds to heparan sulfate glycosaminoglycan. Heparin (heparin), De2SHep (2-O-desulfated heparin), De6SHep (6-O-desulfated heparin), DeNSHep (N-desulfated heparin) and DeNS/AcHep (N-desulfated/acetylated heparin).

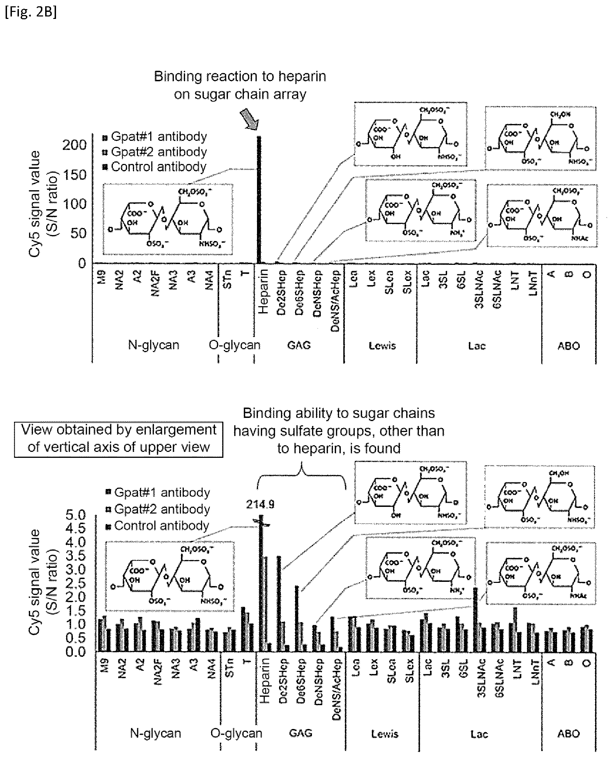

Fig. 2B shows the binding of a Cy5-labeled Gpat#1 antibody and a Gpat#2 antibody to N-glycan (M9, NA2, A2, NA2F, NA3, A3 and NA4), O-glycan (STn and T), GAG (heparin, De2SHep, De6SHep, DeNSHep and DeNS/AcHep), Lewis antigens (Lea, Lex, SLea and SLex), Lac (Lac, 3SL, 6SL, 3SLNAc, 6SLNAc, LNT and LNnT) and ABO antigens (A, B and O). In the upper view and the lower view, the same experimental results are shown by changing the numerical values of the vertical axis indicating Cy5 signal values. In the views, the structures of principal repeating units of heparin, De2SHep, De6SHep, DeNSHep and DeNS/AcHep are shown.
Fig. 2C shows, according to aggregation of FITC fluorescence signals, that Protein-G magnetic bead-bound Gpat#1 antibody and Gpat#2 antibody bind to FITC-conjugated heparin.
Fig. 3A shows FITC signals in a flow cytometer, after the FITC-labeled antibodies of the present invention have been added to the culture media of the gastric cancer cell lines NUGC3 and Katolll, the pancreatic cancer cell line Panc1, the liver cancer cell line HuH7, and the lung cancer cell line NCI-H2170, followed by performing culture.
Fig. 3B shows internalization of the FITC-labeled Gpat#1 antibody and Gpat#2 antibody of the present invention into Katolll and NUGC3 cells.
Fig. 4A shows the results of an MTT assay, in which the Gpat#1 antibody and the Gpat#2 antibody were used in the gastric cancer cell lines Katolll, NUGC3, HGC27 and MKN1. The results of independent four experiments are shown by an average and a standard deviation. The asterisk indicates p < 0.05.
Fig. 4B shows the results of an MTT assay, in which the Gpat#1 antibody and the Gpat#2 antibody were used in the pancreatic cancer cell lines (Panc1, AsPC1 and Capan1), the colon cancer cell lines (SW480, SW620 and HCT116), the liver cancer cell lines (HepG2, HuH7, Alex and HLF), and the lung cancer and malignant pleural tumor (HCI-H2170, A549 and NCI-H28). The results of independent four experiments are shown by an average and a standard deviation. The asterisk indicates p < 0.05.
Fig. 4C shows the results of an MTT assay, which was carried out on the gastric cancer cell line NUGC3 in the absence of heparin (heparin (-)) or in the presence of heparin (heparin (+): 60 µg/ml; and heparin (++): 100 µg/ml). The results of independent four experiments are shown by an average and a standard deviation. The asterisk indicates p < 0.05.
Fig. 5 shows the cell growth inhibitory effects of the combined use of the antibody of the present invention and an anticancer agent-conjugated secondary antibody on the gastric cancer cell lines Katolll and NUGC3.

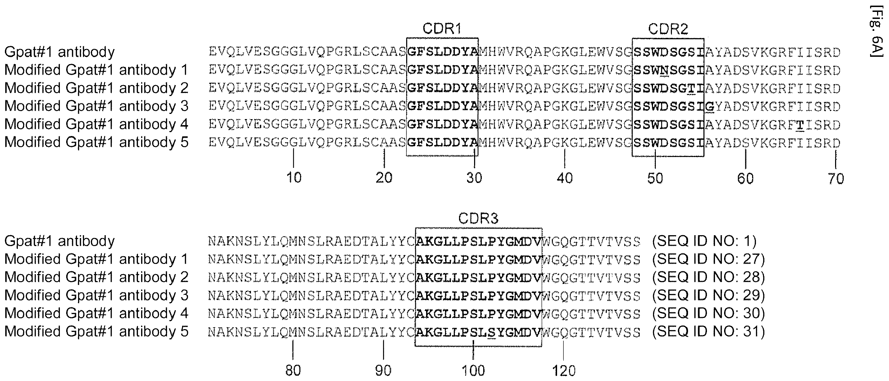

Fig. 6A shows the amino acid sequences (SEQ ID NO: 27 to 31) of the heavy chains of 5 types of antibodies (a modified Gpat#1 antibody 1 to a modified Gpat#1 antibody 5) obtained by modifying the amino acid sequence (SEQ ID NO: 1) of the Gpat#1 antibody, which are in comparison with the sequence set forth in SEQ ID NO: 1. CDR1 to CDR3 of each antibody are indicated with the bold letters and the square, and the amino acid residues in the sequences of the modified antibodies 1 to 5 that are different from those in SEQ ID NO: 1 are indicated with the underline. The numbers shown below the sequences correspond to the positions of amino acids in SEQ ID NOS: 1 and 27 to 31.

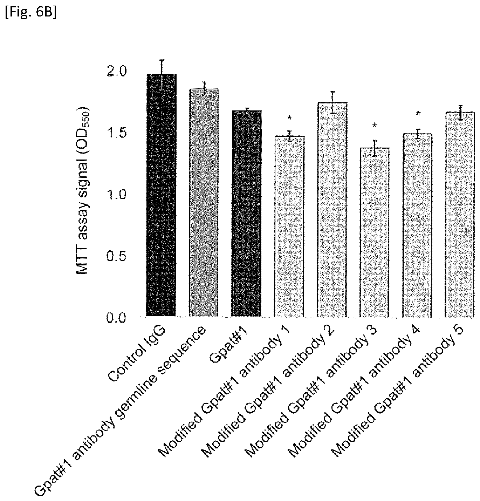

Fig. 6B shows the results of an MTT assay of using the modified Gpat#1 antibody 1 to the modified Gpat#1 antibody 5, which are in comparison to control IgG, Gpat#1 germline sequence, and Gpat#1 antibody. The results of independent four experiments are shown by an average and a standard deviation. Antibodies whose cell growth inhibitory effects were significantly increased (p < 0.05), when compared with the Gpat#1 antibody, are indicated with the asterisk. The "Gpat#1 germline sequence" antibody is an antibody, in which a V region constituting the Gpat#1 antibody is specified with the amino acid sequence of a germ cell gene without somatic hypermutation (wherein the IGHV3-9^{∗}01 sequence is obtained from IMGT database: http://www.imgt.org/).

### Detailed Description

The present invention provides an antibody that binds to sulfated glycosaminoglycan and has cell growth inhibitory activity.

### < Sulfated glycosaminoglycan >

Glycosaminoglycan is a long linear polysaccharide having repeating disaccharide units, wherein the repeating unit is composed of an amino sugar along with uronic acid or galactose. Hyaluronic acid, chondroitin sulfate, dermatan sulfate, keratan sulfate, heparan sulfate, and heparin have been known as naturally occurring glycosaminoglycans.

Hyaluronic acid has a repeating disaccharide unit structure that is constituted with D-glucosamine and D-glucuronic acid; chondroitin sulfate has a repeating disaccharide unit structure that is constituted with D-galactosamine and D-glucuronic acid; dermatan sulfate has a repeating disaccharide unit structure that is constituted with D-galactosamine and L-iduronic acid; keratan sulfate has a repeating disaccharide unit structure that is constituted with D-glucosamine and D-galactose; and heparan sulfate and heparin each have a repeating disaccharide unit structure that is constituted with D-glucosamine serving as amino acid and L-iduronic acid or D-glucuronic acid serving as uronic acid. In the present description, the amino group of amino sugar, namely, D-glucosamine or D-galactosamine, of glycosaminoglycan may be or may not be acetylated. In addition, glycosaminoglycan may be or may not be sulfated.

Glycosaminoglycan having a molecular weight in the range of 3,000 to 30,000 binds to a protein that is expressed in various tissues of a living body, for example, on the cell surface, so that it is present as proteoglycan. The function of glycosaminoglycan in a living body has not yet been elucidated in many respects.

The term "sulfated glycosaminoglycan" is used in the present description to mean glycosaminoglycan having 1 to 4 sulfate groups per disaccharide unit. Herein, the sulfate group means SO₃⁻ that is introduced to replace for hydrogen of a hydroxyl group or an amino group on amino sugar and/or uronic acid and/or galactose.

Hence, hyaluronic acid having no sulfate groups in nature is not included in sulfated glycosaminoglycan. However, in the present description, sulfated glycosaminoglycan includes not only naturally occurring sulfated glycosaminoglycan, but also includes glycosaminoglycan into which sulfate group(s) are artificially introduced. Thus, the present sulfated glycosaminoglycan includes, for example, natural hyaluronic acid or synthetic glycosaminoglycan, which comprises one or more sulfate groups, on average, per disaccharide unit.

Herein, the phrase "on average, per disaccharide unit" may be used to mean a numerical value obtained by dividing the number of sulfate groups in the entire glycosaminoglycan molecules in glycosaminoglycan having a repeating disaccharide unit structure, by the number of disaccharide units. As such, for example, the phrase "three or more, on average, per disaccharide unit" is used to mean that 2.5 to 3.4 sulfate groups are comprised per disaccharide unit, when the number of sulfate groups in the entire glycosaminoglycan molecules is divided by the number of disaccharide units.

However, since the epitopic site recognized by an antibody is a partial structure further limited in glycosaminoglycan, the average number of sulfate groups per disaccharide unit may be in the aforementioned range in some regions of glycosaminoglycan.

### < Heparan sulfate glycosaminoglycan >

The above-described sulfated glycosaminoglycan includes heparan sulfate glycosaminoglycan. The term "heparan sulfate glycosaminoglycan" used in the present description includes both heparin, in which heparin having a low molecular weight is used as an anticoagulant, and heparan sulfate glycosaminoglycan in a narrow sense, having a structure similar to heparin.

### < Anti-sulfated glycosaminoglycan antibody >

The antibody of the present invention can specifically recognize sulfated glycosaminoglycan and can bind thereto. For example, the antibody of the present invention binds to glycosaminoglycan having one or more sulfate groups, on average, per disaccharide unit, but does not bind to glycosaminoglycan having no sulfate groups. Moreover, the antibody of the present invention binds to glycosaminoglycan having two or more sulfate groups, on average, per disaccharide unit, but does not bind to glycosaminoglycan having one or less sulfate group on average. Otherwise, the antibody of the present invention binds to glycosaminoglycan having three or more sulfate groups, on average, per disaccharide unit, but does not bind to glycosaminoglycan having two or less sulfate groups, on average.

In other words, sulfated glycosaminoglycan, to which the antibody of the present invention binds, has one or more, two or more, or three or more sulfate groups, on average, per disaccharide unit.

The antibody of the present invention may be, for example, an antibody, which binds to heparin having three sulfate groups, on average, per disaccharide unit, and desulfated heparin having two sulfate groups, on average, per disaccharide unit (e.g., 2-O-desulfated heparin, 6-O-desulfated heparin, N-desulfated heparin and/or N-desulfated/acetylated heparin), and which has particularly high binding affinity to heparin. A particularly preferred example of the antibody of the present invention may be an antibody specifically recognizing heparin and binding thereto.

Furthermore, the antibody of the present invention binds to heparan sulfate glycosaminoglycan including heparin, but does not bind to: N-glycan such as M9, NA2, A2, NA2F, NA3, A3 and NA4; O-glycan such as STn and T; Lewis antigens such as Lea, Lex, SLea and SLex; Lac such as Lac, 3SL, 6SL, 3SLNAc, 6SLNAc, LNT and LNnT; and ABO antigens such as A, B and O. Accordingly, the antibody of the present invention may be, for example, an antibody that can recognize the above-described repeating structure characteristic for heparan sulfate glycosaminoglycan, or a three-dimensional structure formed from the repeating structure.

The presence or absence of the binding of an antibody to sulfated glycosaminoglycan can be confirmed, for example, by allowing natural sulfated glycosaminoglycan to come into contact with, for example, an antibody labeled with a fluorescent label or the like under conditions commonly used in the present technical field, and then detecting signals from the label. For example, as described in Examples later, the binding intensity can be obtained by allowing an antibody fluorescently labeled with Cy5 to come into contact with a substance that is likely to be an antigen, and then by obtaining information of the amount of the antibody bound to the substance in the form of fluorescence intensity or S/N ratio. Otherwise, the presence or absence of the binding of the antibody to sulfated glycosaminoglycan can also be confirmed by allowing natural sulfated glycosaminoglycan to come into contact with the antibody of the present invention, and further, with a labeled secondary antibody capable of recognizing the antibody of the present invention, and then detecting signals from the labeled secondary antibody. The antibody of the present invention may also bind to a modified or synthetic sulfated glycosaminoglycan with a molecular weight in the range of 3,000 to 30,000 that is used as an antigen, instead of natural sulfated glycosaminoglycan.

Besides, in the present description, the terms "to bind" and "to specifically bind" may mean that the binding of an antibody to an antigen has binding affinity with a KD value of 10⁻⁸ M or less, preferably 10⁻⁹ M or less, and more preferably 10⁻¹⁰ M or less, but the meaning of the terms is not particularly limited thereto. Accordingly, the antibody of the present invention is characterized in that it has the above-described binding affinity to sulfated glycosaminoglycan, but does not have the binding affinity to other antigens including other sugars and proteins.

Moreover, in the present description, the phrase "to specifically bind to a target substance" may mean that the binding of the present antibody to a target substance such as, for example, sulfated glycosaminoglycan, heparan sulfate glycosaminoglycan, or heparin, is two times or more, three times or more, or four times or more stronger than, for example, the binding of the present antibody to a substance other than a target substance in a case where an excessive amount of target substance is used, and detection is then carried out according to a common binding assay. Furthermore, the phrase "to specifically bind to a target substance" may mean that the S/N ratio is 2 or more, 3 or more, or 4 or more, when the binding is detected using a fluorescent label, as described above.

The antibody of the present invention inhibits the growth of cells having sulfated glycosaminoglycan, through the binding thereof to the sulfated glycosaminoglycan. This activity can be detected in the absence of an effector cell and a complement, and thus, this activity can be distinguished from antibody-dependent cellular cytotoxicity (ADCC) and complement-dependent cytotoxicity (CDC). However, when the antibody of the present invention is used as a cell growth inhibitor and a targeting reagent for drug delivery, it should be understood that such an effector cell and a complement may be present. As understood in the present technical field, the Fc region of an antibody has been known to have effector function, and the Fc region can provide activation of complements and activation of effector cells. When such a complement is activated, it can dissolve cells binding to variable regions. Examples of the "effector cells" may include killer T cells, helper T cells, natural killer cells, and macrophages. The Fc receptor present on the surface of such an effector cell binds to the Fc region of an antibody and is thereby activated, so that it can exhibit cytotoxicity.

The cell growth inhibitory activity of the antibody of the present invention can be measured, for example, by an MTT assay or the like, after the antibody has been cultured together with cells of interest. When 1.0 to 4.0 µg of the antibody is used to, for example, 1.0 x 10³ to 1.0 x 10⁴ cells, such as cancer cells, the growth of the cells can be inhibited at a percentage of 10% or more, 20% or more, or 30% or more, without any limitations.

The antibody of the present invention may be either a polyclonal antibody or a monoclonal antibody, as long as it binds to sulfated glycosaminoglycan. The present antibody is preferably a monoclonal antibody. In addition, the antibody of the present invention may be any of non-human antibodies, such as the antibodies of mice, rabbits, goats and the like, chimeric antibodies, humanized antibodies, and human antibodies. When the antibody is used in the treatment of human diseases such as cancer, it is preferably a humanized antibody or a human antibody, although the present antibody is not particularly limited thereto. In view of the foregoing, the antibody that can be preferably used in the present invention may be an antibody comprising the framework region (FR) of a human antibody and a complementarity determining region (CDR) capable of providing high binding affinity to sulfated glycosaminoglycan.

The antibody of the present invention can be obtained in the form of a polyclonal antibody or a monoclonal antibody, by immunizing a non-human mammal with sulfated glycosaminoglycan that is specified as an antigen, and then performing a known method. The antibody of the present invention can also be obtained by performing a genetic engineering method, based on the amino acid sequence information of the antibody of the present invention with demonstrated activity, or the nucleotide sequence information of a polynucleotide encoding the aforementioned antibody, or by synthesizing it according to a chemical synthetic method.

When the antibody is produced according to a genetic engineering method, polynucleotides encoding a heavy chain and a light chain are introduced into a suitable host cells and are then allowed to express therein, so that the antibody can be obtained in the form of a recombinant protein. In this case, the polynucleotide may be either DNA or RNA, and as a method of introducing the polynucleotide into host cells, a method used in the present technical field can be appropriately utilized. As a vector for introducing the polynucleotide into host cells, a viral vector, a plasmid vector, a phage vector, etc. can be appropriately used. Examples of the host cells that can be used herein may include bacteria such as *Escherichia coli,* yeasts, insect cells, and animal cells. Herein, the polynucleotides encoding a heavy chain and a light chain may be introduced into individual vectors, separately, or the polynucleotides may be connected with each other and may be introduced into a single vector.

For example, as described in the Examples below, cDNAs encoding the heavy chain and light chain of the antibody of the present invention are incorporated into a vector optionally comprising a signal sequence, a poly A sequence, further, a control sequence such as a promoter sequence, and a selective marker, and thereafter, the vector is introduced into suitable host cells, followed by culturing the host cells, so that an antibody capable of specifically recognizing sulfated glycosaminoglycan can be obtained in the form of a recombinant protein.

When the antibody of the present invention is used as a monoclonal antibody, the antibody may be an IgG antibody molecule, or an antigen-binding fragment thereof, or an antigen-binding derivative thereof. For example, the antibody may be a complete antibody, Fab, Fab', a F(ab')₂ fragment, or a single chain antibody (scFv) fragment produced by linking a heavy chain variable region (VH) and a light chain variable region (VL) via a linker, scFv-Fc, sc(Fv)₂, Fv, a diabody, etc.

The aforementioned scFv, scFv-Fc, and sc(Fv)₂ are synthetic peptides produced by linking variable regions to each other via a linker. The linker is not particularly limited, as long as it is commonly used in the present technical field. For example, a peptide linker consisting of 5 to 25, and preferably 10 to 20 amino acid residues, such as a GS linker, can be preferably used.

The antibody of the present invention further includes derivatives that can be understood by a person skilled in the art, such as, for example, derivatives that have been modified to facilitate antibody purification or to enhance stability, and complexes formed by binding the present antibody to drugs such as anticancer agents, within a range in which the derivatives do not affect the antigen-binding ability of the antibody. In the present description, fragments and derivatives retaining their binding ability to sulfated glycosaminoglycan are intended to be included in the "antibody" for convenience, as long as there is no contradiction in the context.

The antibody of the present invention can also be synthesized in the form of a multimer such as a dimer, a trimer, or a tetramer. Further, the antibody of the present invention may be a bi-specific antibody having first specificity of binding to sulfated glycosaminoglycan and second specificity of binding to another antigen. The second specificity may be either specificity directed towards, for example, another antigen that can be expressed by target cells, or specificity directed towards a drug such as an anticancer agent, and thus, it can be appropriately selected depending on purpose. When the second specificity is directed towards another antigen that can be expressed by target cells, for example, by cancer cells, as a result of the use of such a bi-specific antibody, the delivery of the antibody to the target cells is more specifically carried out. On the other hand, when the second specificity is directed towards a drug such as an anticancer agent, the drug, together with the bi-specific antibody, can be delivered in the form of a complex to a target site.

A person skilled in the art could obtain the antibody of the present invention in an appropriate form depending on intended use, based on the present description and common general knowledge in the present technical field.

Examples of the antibody of the present invention that binds to sulfated glycosaminoglycan may include, but are not particularly limited to, the following antibodies that bind to heparin:
(1) an antibody having a heavy chain variable region comprising CDR1 consisting of the amino acid sequence set forth in SEQ ID NO: 13, CDR2 consisting of the amino acid sequence set forth in SEQ ID NO: 14, and CDR3 consisting of the amino acid sequence set forth in SEQ ID NO: 15;
(2) an antibody having a light chain variable region comprising CDR1 consisting of the amino acid sequence set forth in SEQ ID NO: 16, CDR2 consisting of the amino acid sequence set forth in SEQ ID NO: 17, and CDR3 consisting of the amino acid sequence set forth in SEQ ID NO: 18;
(3) an antibody having a heavy chain variable region comprising CDR1 consisting of the amino acid sequence set forth in SEQ ID NO: 19, CDR2 consisting of the amino acid sequence set forth in SEQ ID NO: 20, and CDR3 consisting of the amino acid sequence set forth in SEQ ID NO: 21; and
(4) an antibody having a light chain variable region comprising CDR1 consisting of the amino acid sequence set forth in SEQ ID NO: 22, CDR2 consisting of the amino acid sequence set forth in SEQ ID NO: 23, and CDR3 consisting of the amino acid sequence set forth in SEQ ID NO: 24.

Moreover, examples of the antibody of the present invention that binds to sulfated glycosaminoglycan may include the following antibodies that bind to heparin:
(5) an antibody having a heavy chain variable region having the amino acid sequence set forth in SEQ ID NO: 1 or 4, and a light chain variable region having the amino acid sequence set forth in SEQ ID NO: SEQ ID NO: 2 or 6; and
(6) an antibody having a heavy chain variable region having the amino acid sequence set forth in SEQ ID NO: 7 or 10, and a light chain variable region having the amino acid sequence set forth in SEQ ID NO: SEQ ID NO: 8 or 12.

Furthermore, examples of the antibody of the present invention that binds to sulfated glycosaminoglycan may include the following antibodies that bind to heparin:
(7) an antibody having a heavy chain variable region consisting of an amino acid sequence having a sequence identity of 90% or more, for example, 90%, 91%, 92%, 93%, 94%, 95%, 96%, 97%, 98%, or 99% to SEQ ID NO: 1, and a light chain variable region consisting of an amino acid sequence having a sequence identity of 90% or more, for example, 90%, 91%, 92%, 93%, 94%, 95%, 96%, 97%, 98%, or 99% to SEQ ID NO: 2; and
(8) an antibody having a heavy chain variable region consisting of an amino acid sequence having a sequence identity of 90% or more, for example, 90%, 91%, 92%, 93%, 94%, 95%, 96%, 97%, 98%, or 99% to SEQ ID NO: 7, and a light chain variable region consisting of an amino acid sequence having a sequence identity of 90% or more, for example, 90%, 91%, 92%, 93%, 94%, 95%, 96%, 97%, 98%, or 99% to SEQ ID NO: 8.

Further, an example of the antibody of the present invention that binds to sulfated glycosaminoglycan may be the following antibody that binds to heparin:
(9) an antibody having a heavy chain and/or a light chain each consisting of an amino acid sequence comprising a substitution, deletion, and/or addition of one or several amino acids in the heavy chain variable regions and/or the light chain variable regions of the antibodies (1) to (6).

Herein, the term "several" may mean 5 or less, for example, 1, 2, 3, 4, or 5. Accordingly, the antibody of the present invention may have a modification of 5 or less amino acids in each of the heavy chain variable regions and/or the light chain variable regions of the antibodies (1) to (6). An antibody that is functionally equivalent to a certain antibody can be prepared according to a method well known in the present technical field, such as, for example, site-directed mutagenesis.

The binding properties of an antibody are basically determined by the CDRs of a heavy chain and a light chain thereof. Accordingly, from the viewpoint of less influence on binding properties, the modification is preferably in a framework region or in a constant region. Substitution is not particularly limited, but it may be conservative substitution. Moreover, it is preferable that the modification preferably does not largely change the three-dimensional structure of the antibody.

However, it has been known that after generation of an antibody reacting against a certain antigen in a living body, an antibody having more improved affinity to the certain antigen than the original antibody is generated according to a process called "affinity maturation." Also, according to the findings by the present inventors, it is found that variously mutated antibody molecules are generated in tumor infiltrating B cell clones by somatic hypermutation. Accordingly, on the basis of information regarding the amino acid sequences and nucleotide sequences of the antibodies as specifically disclosed above, it is possible to obtain antibodies each having activity equivalent to or higher than the disclosed antibodies, more specifically, antibodies having binding affinity to sulfated glycosaminoglycan, cell growth inhibitory activity, and functionality as a targeting reagent for drug delivery, which are more improved than the disclosed antibodies. In fact, as described in the Examples later, the present inventors have confirmed that the antibodies that have been modified based on the disclosed sequences may exhibit more improved cell growth inhibitory activity than the antibodies before the modification. These modifications may occur in the CDR region, as well as in the framework region and the constant region.

Hence, the antibody of the present invention may also have a modification caused by a substitution, deletion, and/or addition in the CDR regions of the sequences specifically disclosed in the present description.

Examples of the thus modified antibody may include the following antibody of the present invention that binds to sulfated glycosaminoglycan, specifically to heparin:
(10) the antibody according to the above (9), having one or more substitutions selected from the substitution of the amino acid at position 51 Asp with Asn, the substitution of the amino acid at position 56 Ala with Gly, and the substitution of the amino acid at position 66 Ile with Thr, in the amino acid sequence set forth in SEQ ID NO: 1.

The above-described antibodies having three types of modifications exhibited more improved cell growth inhibitory activity than that of the antibody before the modification. Moreover, an antibody having the substitution of the amino acid at position 54 Ser with Thr or the substitution of the amino acid at position 102 Pro with Ser in SEQ ID NO: 1 also exhibited cell growth inhibitory activity equivalent to that of the antibody before the modification. Therefore, for example, an antibody comprising 1, 2, 3, 4, or 5 types of substitutions selected from the substitution of the amino acid at position 51 Asp with Asn, the amino acid at position 54 Ser with Thr, the substitution of the amino acid at position 56 Ala with Gly, the substitution of the amino acid at position 66 Ile with Thr, and the substitution of the amino acid at position 102 Pro with Ser, in the heavy chain variable region having the amino acid sequence set forth in SEQ ID NO: 1, can also be preferably used as the antibody of the present invention.

Besides, regarding the above-described substitutions, the substitution of the amino acid at position 51 Asp with Asn, the amino acid at position 54 Ser with Thr, and the substitution of the amino acid at position 56 Ala with Gly are considered to be conservative substitutions. On the other hand, the substitution of the amino acid at position 66 Ile with Thr and the substitution of the amino acid at position 102 Pro with Ser are considered to be non-conservative substitutions.

### < Cell growth inhibitor >

The present invention also provides a cell growth inhibitor comprising an antibody binding to sulfated glycosaminoglycan as an active ingredient. The antibody that binds to sulfated glycosaminoglycan is as described above. The cell growth inhibitor of the present invention can be used in combination with another drug having cell growth inhibitory activity. In addition, the cell growth inhibitor of the present invention can be used in combination with a drug having cytotoxicity. It has been confirmed that the antibody of the present invention binds to a cell having sulfated glycosaminoglycan and is then internalized into the cell. Accordingly, it is anticipated that a drug used in combination with the cell growth inhibitor of the present invention, for example, a drug having cytotoxicity will be internalized into a cell and will exhibit cytotoxic activity therein. The cell growth inhibitor of the present invention and the drug having cytotoxic activity can be contacted with a cell, simultaneously or separately. Moreover, the cell growth inhibitor of the present invention can also be used in combination with another anticancer therapy such as radiation therapy.

Examples of the drug having cell growth inhibitory activity or cytotoxicity may include, but are not limited to, proteins such as toxin, antimitotics, alkylating agents, antibiotics, antimetabolites, apoptotic drugs, alkaloid agents, anticancer agents such as Taxoid, and radioisotopes. Examples of the toxin may include diphtheria toxin, saporin toxin, Pseudomonas aeruginosa toxin, abrin toxin, ricin A toxin, tumor necrosis factor, and interferon-y. Examples of the anticancer agent may include, but are not limited to, molecular target drugs such as cyclophosphamide, dacarbazine, cisplatin, carboplatin, methotrexate, fluorouracil, crizotinib, sunitinib, sorafenib, afatinib, lapatinib, imatinib, erlotinib or everolimus, monomethyl auristatin E (MMAE), monomethyl auristatin F (MMAF), duocarmycin (DM), dolastatin (MD10), amanitin (AAMT), and PNU-159682 (nemorubicin metabolite). Examples of the radioisotopes may include ⁹⁰Y and ¹³¹I.

These drugs can be used by being conjugated with the antibody of the present invention. The method of connecting the antibody with the drug is not limited, and the antibody preferably binds to the drug, for example, via covalent bond. The drug may be added to the antibody, for example, via a linker. The linker may be may be any given substance that can constitute a linking portion between a drug and an antibody, such as a peptide, fatty acid, or fatty acid ester. The linker may be inserted between a drug and an antibody, as a spacer for not inhibiting the three-dimensional structure of the antibody, or as a cleavable linking portion comprising a protease recognition site. The linking of a drug to the antibody of the present invention is preferably carried out at a site of the antibody that does not impede the binding of the antibody to sulfated glycosaminoglycan, for example, at the Fc region of the antibody. The linking of a drug to the antibody may be cleaved after the drug and the antibody have been internalized into target cells.

Moreover, as described above, the antibody of the present invention is provided as a bi-specific antibody having first specificity to sulfated glycosaminoglycan and second specificity to another antigen that can be expressed by target cells, such as, for example, by cancer cells, so that the delivery of the antibody to the target cells can be more specifically carried out, and the antibody can more effectively exhibit cell growth inhibitory activity. Furthermore, when the second specificity of the present antibody is specificity to a drug having cell growth inhibitory activity or cytotoxicity, such as an anticancer agent, the drug, together with the bi-specific antibody, can be delivered as a complex to the target site.

Otherwise, the drug is conjugated to a secondary antibody capable of binding to the antibody of the present invention (i.e., Secondary Antibody-Drug Conjugate), and the conjugate can be used in combination with the antibody of the present invention. The linking of the drug to the secondary antibody can be carried out in the same manner as described above. Alternatively, a secondary antibody linked to a drug can be acquired as a commercially available product.

Specifically, the cell growth inhibitor of the present invention can be used, for example, to inhibit the growth of cancer cells. The cancer cells may be cancer cells in gastric cancer, pancreatic cancer, colon cancer, liver cancer, lung cancer, breast cancer, skin cancer, oral cancer, esophageal cancer, bile duct cancer, endometrial cancer, cervical cancer, ovarian cancer, renal cell carcinoma, urothelial cancer, prostate cancer, and/or malignant pleural tumor.

In the Examples described later, it is demonstrated that when an anticancer agent-conjugated secondary antibody is used in combination with the antibody of the present invention, inhibition of the growth of cancer cells and cell death are induced.

### < Targeting reagent >

The present invention also provides a targeting reagent for drug delivery, comprising an antibody binding to sulfated glycosaminoglycan. The antibody binding to sulfated glycosaminoglycan is as described above. By combining the antibody with the targeting reagent of the present invention, a drug such as an anticancer agent can be delivered to target cells having sulfated glycosaminoglycan.

The target cells are cells, to which not only inhibition of the cell growth, but also cytotoxic effects are intended to be provided by the delivery of a drug such as an anticancer agent. The target cells are, for example, cancer cells. The targeting reagent of the present invention and a drug intended to be delivered to target cells can be administered to a subject having the target cells, simultaneously or separately.

For example, as described above, the antibody of the present invention is provided as a bi-specific antibody having first specificity to sulfated glycosaminoglycan and second specificity to a drug, so that the drug, together with the bi-specific antibody, can be delivered as a complex to a target site.

Otherwise, as described above, a drug is conjugated to a secondary antibody capable of binding to the antibody of the present invention, and the conjugate can be used in combination with the targeting reagent of the present invention.

The targeting reagent of the present invention can also be used, not for the purpose of the cell death of target cells. In this case, for the purpose of avoiding the phenomenon that effector cells and/or compliments are activated in the body of a subject, to which the targeting reagent and the drug have been administered, so that they provide antibody-dependent cellular cytotoxicity (ADCC) and complement-dependent cytotoxicity(CDC), the antibody of the present invention comprised in the targeting reagent is preferably configured not to contain an Fc region.

### < Pharmaceutical composition >

The present invention also provides a pharmaceutical composition comprising, as an active ingredient, an effective amount of the above-described cell growth inhibitor of the present invention or the above-described targeting reagent of the present invention, alone or in combination with another active ingredient. Depending on the administration form, the pharmaceutical composition may comprise pharmaceutically acceptable carriers, excipients, buffers, stabilizers or the like, which are generally used in the present technical field, in addition to the cell growth inhibitor or targeting reagent of the present invention and another active ingredient.

The cell growth inhibitor, targeting reagent or pharmaceutical composition of the present invention can be locally or systemically administered to a subject. Thus, although the administration form is not particularly limited, it is preferably injected or infused, for example, into the vein, the abdominal cavity, the affected area, or the vicinity of the affected area. For instance, by local administration into cancer tissues, the antibody of the present invention and a drug arbitrarily targeted by the antibody of the present invention are accumulated in the cancer tissues, so that cancer cell-specific cell growth inhibitory effects can be more effectively exhibited.

The applied dose of the antibody of the present invention used as an active ingredient changes depending on the patient's body weight and age, the severity of disease, etc. Thus, although the applied dose is not particularly limited, the antibody of the present invention can be administered, for example, in a dose range of 0.0001 to 100 mg/kg of body weight, once to several times a day, every two days, every three days, every week, every two weeks, every month, every two months, or every three months.

### < Detecting agent >

As described above, it has been discovered that an antibody reacting against sulfated glycosaminoglycan infiltrates into human diffuse-type gastric carcinoma tissues, and it has also been confirmed that the antibody of the present invention binds to various human cancer cells. Hence, the present invention also provides an agent for detecting cancer, comprising the antibody of the present invention. The detecting agent of the present invention can be used to detect cancer selected from gastric cancer, pancreatic cancer, colon cancer, liver cancer, lung cancer, breast cancer, skin cancer, oral cancer, esophageal cancer, bile duct cancer, endometrial cancer, cervical cancer, ovarian cancer, renal cell carcinoma, urothelial cancer, prostate cancer, and/or malignant pleural tumor. The detecting agent of the present invention can be used to detect cancer selected from, for example, gastric cancer, pancreatic cancer, colon cancer, liver cancer, lung cancer, and malignant pleural tumor.

Specifically, a biological sample derived from a subject, such as, for example, blood (whole blood, plasma, or serum), lymphatic fluid, interstitial fluid, pleural fluid or a biopsy sample, is allowed to come into contact with the detecting agent comprising the antibody of the present invention, so that the presence or absence of an antigen binding to the antibody of the present invention, namely, the presence or absence of sulfated glycosaminoglycan can be detected. For instance, the antibody of the present invention, or a secondary antibody reacting against the antibody of the present invention is labeled with a fluorescent reagent, a coloring reagent or the like, and the fluorescence or color is then detected, so that the presence or absence of the antigen can be confirmed.

Accordingly, the present invention also provides a method for detecting cancer cells, comprising the above-described steps. The detection method is not limited, and for example, methods known in the present technical field, such as ELISA, immunoprecipitation, or immunohistochemistry, can be utilized.

Further, the detecting agent of the present invention can also be provided in the form of being included in a detection kit comprising a reaction solution for the binding reaction of the antibody to sulfated glycosaminoglycan, a reaction vessel, a labeling reagent for detection, a secondary antibody a buffer, an instruction manual, and the like.

### < Treatment method >

The present invention also provides a method for treating cancer in a subject, comprising administering the above-described cell growth inhibitor of the present invention, the above-described targeting reagent of the present invention, or the above-described pharmaceutical composition of the present invention, to a subject. The method of the present invention comprises administering the cell growth inhibitor, targeting reagent, or pharmaceutical composition of the present invention, to a subject suffering from a disease caused by cells having sulfated glycosaminoglycan.

The subjects as targets of the treatment method of the present invention are non-human animals and humans, and are particularly humans.

### Examples

Hereinafter, the present invention will be described in more detail in the following examples. However, these examples are not intended to limit the scope of the present invention.

The following cell lines were used in the present invention: human gastric cancer cell lines KATOIII, HGC27, MKN1, and NUGC3; human pancreatic cancer cell lines Panc-1, AsPC-1, and Capan-1; human colon cancer cell lines SW480, SW620, and HCT116; human liver cancer cell lines HepG2, HuH7, Alexander, and HLF; and human lung cancer and malignant pleural tumor cell lines NCI-H2170, A549, and NCI-H28. These cell lines were each acquired from Institute of Physical and Chemical Research (RIKEN), JCRB Cell Bank, or ATCC, and were each cultured under suitable conditions before use.

### [Example 1: Preparation of antibodies]

A section with a thickness of 10 µm was produced from each of frozen tumor samples derived from 30 cases of human diffuse-type gastric carcinoma, and thereafter, mRNA was extracted from each section using RNeasy Mini kit (Qiagen).

In order to identify antibodies produced by B cells infiltrating into tumor, with the extracted mRNA used as a template, the heavy chain and light chain genes of a B cell antigen receptor (immunoglobulin) were amplified by employing the Multiplex primer set manufactured by iRepertoire, USA., for each case, and the obtained PCR product was then subjected to next-generation sequence analysis involving 300-bp paired-end reading, employing MiSeq System manufactured by Illumina, USA.

The obtained sequence information was integrated and/or filtered, and thereafter, information regarding the positions of V, D and J segments and CDR regions was obtained (iRepertoire (registered trademark), http://www.irepertoire.com/). Furthermore, using the tool of IMGT (http://www.imgt.org), the alignment of V(D)J,C of an antigen receptor gene formed by gene rearrangement was carried out, and a graph regarding the frequency distribution was prepared for each of the heavy chain and the light chain. As a result, a plurality of B cell and/or plasma cell clones highly dominantly existing in a tumor environment were identified.

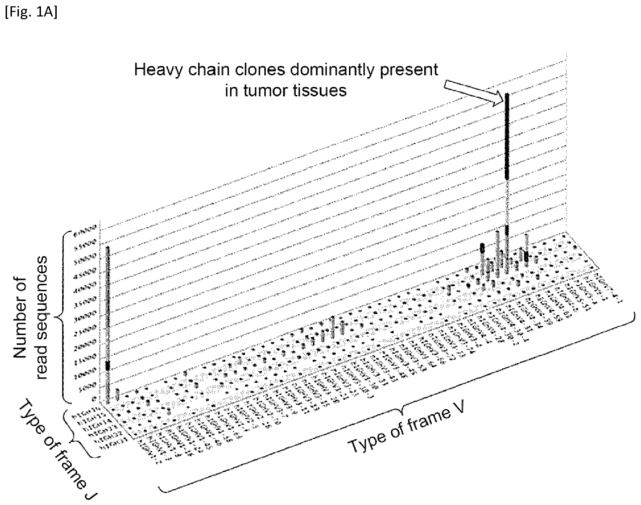

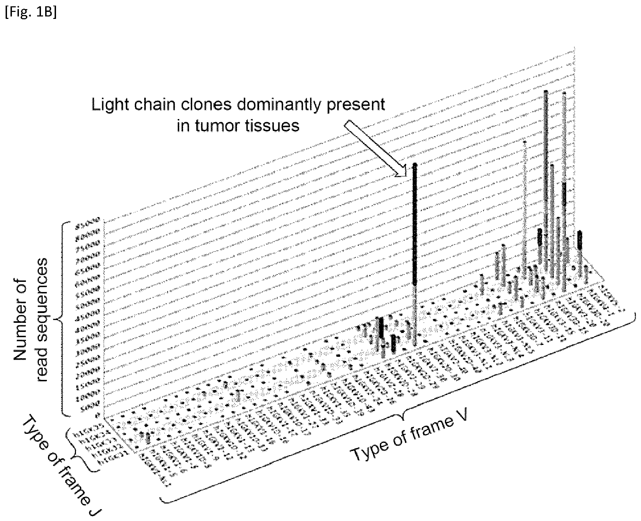

Figs. 1A and 1B each three-dimensionally show a relative frequency distribution of combinations of the V-J genes of heavy chain and light chain (κ) genes encoding antibodies obtained from a single case.

cDNA constructs were produced from heavy chain and light chain genes possessed by the identified clones. Herein, since the nucleotide sequence obtained by the above-described sequence analysis did not contain the 5'-termini of the heavy chain and the light chain, a 5' sequence assumed by the alignment of a V-segment was added to the nucleotide sequence, and at the same time, codon optimization suitable for expression in mammals was carried out. It is to be noted that the thus added 5' sequence encodes a framework region (FW1) that is located closer to the N-terminus than the CDR regions of the heavy chain and the light chain.

The cDNA constructs encoding the heavy chain and the light chain were each incorporated into a pcDNA3 plasmid (Life Technologies), and they were then introduced into HEK293 cells in each clone combination, so that the heavy chain and the light chain were forcibly expressed. Thereafter, purification was carried out, and two types of antibody molecules (Gpat#1 and Gpat#2 antibodies) were reconstructed (ACRO Biosystems).

The Gpat#1 antibody is an IgG antibody that is composed of two heavy chain molecules having a variable region consisting of the amino acid sequence set forth in SEQ ID NO: 1 and two light chain molecules having a variable region consisting of the amino acid sequence set forth in SEQ ID NO: 2. The heavy chain of the Gpat#1 antibody can be obtained by adding a sequence necessary for reconstruction of the antibody to the N-terminus on the basis of the sequence information obtained by the sequence analysis (nucleotide sequence: SEQ ID NO: 3; and amino acid sequence: SEQ ID NO: 4). The light chain of the Gpat#1 antibody can be obtained by adding a sequence necessary for reconstruction of the antibody to the N-terminus on the basis of the sequence information obtained by the sequence analysis (nucleotide sequence: SEQ ID NO: 5; and amino acid sequence: SEQ ID NO: 6).

The Gpat#2 antibody is an IgG antibody that is composed of two heavy chain molecules having a variable region consisting of the amino acid sequence set forth in SEQ ID NO: 7 and two light chain molecules having a variable region consisting of the amino acid sequence set forth in SEQ ID NO: 8. The heavy chain of the Gpat#2 antibody can be obtained by adding a sequence necessary for reconstruction of the antibody to the N-terminus on the basis of the sequence information obtained by the sequence analysis (nucleotide sequence: SEQ ID NO: 9; and amino acid sequence: SEQ ID NO: 10). The light chain of the Gpat#2 antibody can be obtained by adding a sequence necessary for reconstruction of the antibody to the N-terminus on the basis of the sequence information obtained by the sequence analysis (nucleotide sequence: SEQ ID NO: 11; and amino acid sequence: SEQ ID NO: 12).

The amino acid sequences of individual CDRs of the reconstructed Gpat#1 and Gpat#2 antibodies are shown in Table 1.

**[Table 1]**

| | | CDR1 | CDR2 | CDR3 |
|---|---|---|---|---|
| Gpa1# 1 | Heavy chain | GFSLDDYA (SEQ ID NO: 13) | SSWDSGSI (SEQ ID NO: 14) | AKGLLPSLPYGMDV (SEQ ID NO: 15) |
| | Light chain | QNIFYSSNNDNY (SEQ ID NO: 16) | RAS (SEQ ID NO: 17) | QQYYSTPYT (SEQ ID NO: 18) |
| Gpa1# 2 | Heavy chain | GASIRSTAFY (SEQ ID NO: 19) | VYQSGTS (SEQ ID NO: 20) | ASSQAYYDVSGGPVNN (SEQ ID NO: 21) |
| | Light chain | QSLVYTDGNTY (SEQ ID NO: 22) | KVS (SEQ ID NO: 23) | IQGTHWPP (SEQ ID NO: 24) |

### [Example 2: Identification of antigens]

Identification of antigens recognized by the Gpat#1 and Gpat#2 antibodies reconstructed in Example 1 was carried out. First, the possibility of various protein antigens was examined, but protein antigens binding to these antibodies could not be identified.

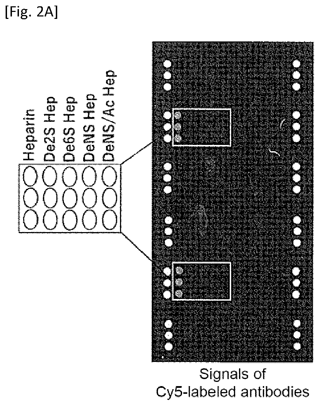

Subsequently, the possibility of sugar antigens was examined. The Gpat#1 and Gpat#2 antibodies were fluorescently labeled with Cy5, using Lightning-Link ^{(R)} Rapid Cy5 conjugation kit (Innova Biosciences), and were then reacted on a sugar chain-immobilized array (BS-X1731) manufactured by Sumitomo Bakelite Co., Ltd., so that Cy5 fluorescent signals were detected. Fig. 2A shows a part of the results of detection of the Gpat#1 antibody, and it is clearly shown that the Gpat#1 antibody particularly binds to heparin, among 5 types of glycosaminoglycans immobilized on the array.

Fig. 2B shows the binding of the Gpat#1 antibody and the Gpat#2 antibody to N-glycan (M9, NA2, A2, NA2F, NA3, A3 and NA4), O-glycan (STn and T), glycosaminoglycan (heparin, De2SHep, De6SHep, DeNSHep and DeNS/AcHep), Lewis antigens (Lea, Lex, SLea and SLex), lactose-containing sugar chains (Lac, 3SL, 6SL, 3SLNAc, 6SLNAc, LNT and LNnT) and ABO antigens (A, B and O). From the results shown in Fig. 2B, it was demonstrated that the Gpat#1 and Gpat#2 antibodies specifically bind to sulfated glycosaminoglycan molecules, in particular, to heparin molecules, among various sugar chains present on the array, and it was identified that the antigen molecules of these antibodies are sulfated glycosaminoglycan.

As shown in Fig. 2B, the Gpat#1 antibody exhibited a binding signal value (S/N ratio) to heparin that was two or more times stronger than the binding thereof to DeNSHep (N-desulfated heparin), and thus, in particular, the Gpat#1 antibody strongly bound to heparin. On the other hand, the Gpat#2 antibody bound to heparin having three sulfate groups per disaccharide unit, in an amount that was three or more times larger than to desulfated heparin (De2SHep) having two sulfate groups per disaccharide unit.

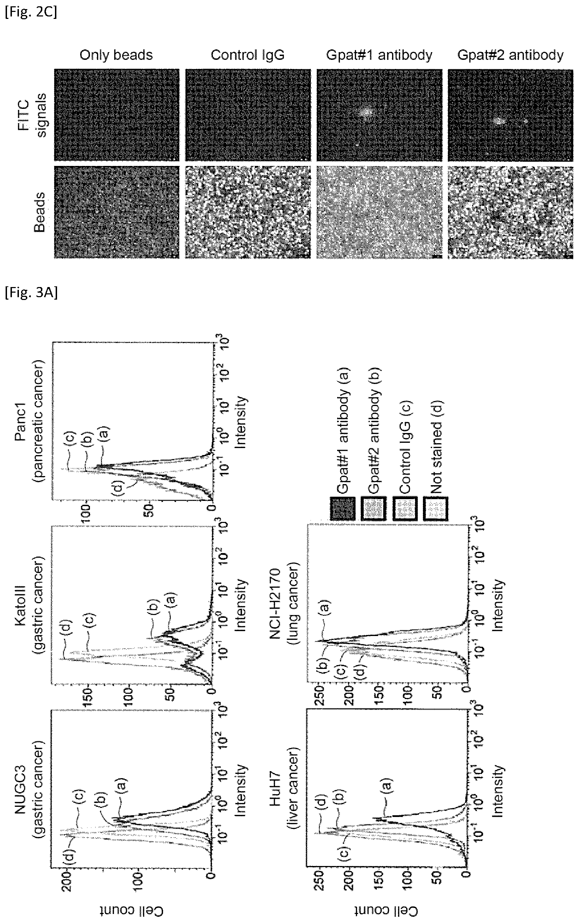

Moreover, the Gpat#1 antibody and the Gpat#2 antibody were each allowed to bind to Protein-G Magnetic Bead (BioRad, USA), and were then mixed with FITC-Conjugated Heparin (Life Technologies, USA), followed by performing a reaction. The resulting beads were washed three times, and were then observed under a fluorescence microscope. As a result, an aggregate of FITC fluorescence signals was observed in the beads, to which the Gpat#1 antibody and the Gpat#2 antibody were allowed to bind, as a result of the binding reaction to the antibodies, and the binding of heparin to the Gpat#1 antibody and the Gpat#2 antibody was confirmed (Fig. 2C).

Thus, in two different types of experimental systems, it was demonstrated that the Gpat#1 antibody and the Gpat#2 antibody bind to sulfated glycosaminoglycan, in particular, to heparin.

### [Example 3: Recognition of cancer cells and internalization of antibodies into cancer cells]

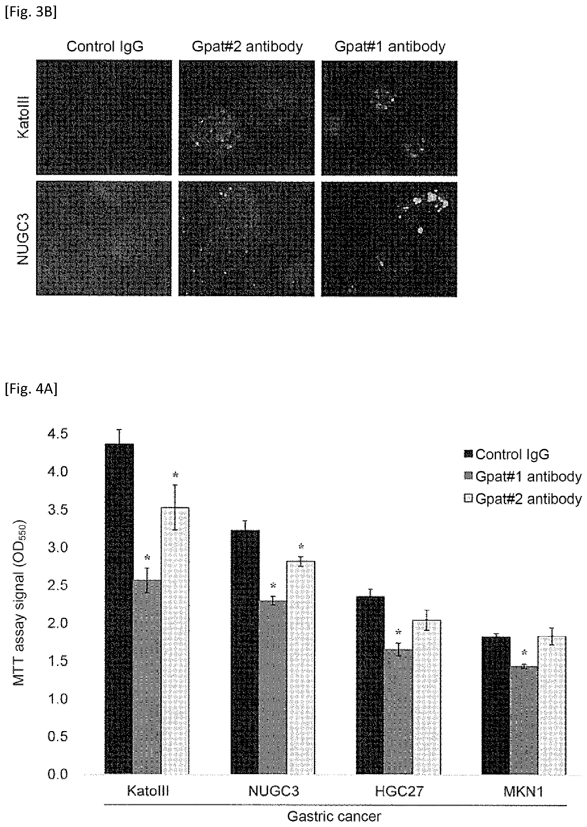

The Gpat#1 antibody and the Gpat#2 antibody obtained in Example 1 were labeled with FITC, and were then added to the culture media of the gastric cancer cell lines NUGC3 and Katolll, the pancreatic cancer cell line Panc1, the liver cancer cell line HuH7, and the lung cancer cell line NCI-H2170, followed by performing a culture at 37°C overnight. The FITC signals were detected using a flow cytometer (MoFlo XDP, Beckman Coulter), and it was confirmed that these antibodies recognize various human cancer cells and bind thereto (Fig. 3A). It was also confirmed by observation under a fluorescence microscope that the antibodies do not only recognize human cancer cells and bind thereto, but are internalized into the cells (internalization) after the binding thereof to the cells (Fig. 3B). Besides, control IgG is another antibody possessed by the present inventors, which is revealed to bind to another protein in the cells. It was confirmed that the control IgG has almost no or completely no reactivity with the above-described cancer cells. The sequences of the heavy chain and light chain variable regions of the used control IgG are shown in SEQ ID NOS: 25 and 26, respectively.

### [Example 4: Verification of cell growth inhibitory effects by MTT assay]

Using the Gpat#1 antibody and the Gpat#2 antibody, 4 types of gastric cancer cell lines (Katolll, NUGC3, HGC27 and MKN1), and also, pancreatic cancer cell lines (Panc1, AsPC1 and Capan1), colon cancer cell lines (SW480, SW620 and HCT116), liver cancer cell lines (HepG2, HuH7, Alex and HLF), and lung cancer and malignant pleural tumor (HCI-H2170, A549 and NCI-H28) were subjected to an MTT assay, by employing Cell Proliferation Kit I (MTT) (Roche Diagnostics).

Katolll (1.0 x 10⁴ cells), NUGC3 (6.0 x 10³ cells), HGC27 (5.0 x 10³ cells), MKN1 (5.0 x 10³ cells), Panc1 (6.0 x 10³ cells), AsPC1 (8.0 x 10³ cells), Capan1 (1.0 x 10⁴ cells), SW480 (8.0 x 10³ cells), SW620 (8.0 x 10³ cells), HCT116 (8.0 x 10³ cells), HepG2 (1.0 x 10⁴ cells), HuH7 (6.0 x 10³ cells), Alex (6.0 x 10³ cells), HLF (7.0 x 10³ cells), HCI-H2170 (6.0 x 10³ cells), A549 (5.0 x 10³ cells), and NCI-H28 (1.0 x 10⁴ cells) were each seeded in a 96-well plate, and the cells were then cultured together with 20 µg/ml (2.0 µg per well) control IgG (human IgG (normal), Invitrogen), the Gpat#1 antibody, or the Gpat#2 antibody, at 37°C for 4 days. The culture was carried out in a D-MEM or RPMI medium, depending on the cell line, while adding FBS (Sigma-Aldrich), penicillin/streptomycin (Wako Pure Chemical Industries), L-glutamic acid (Wako Pure Chemical Industries) and sodium pyruvate (Thermo Fisher Scientific) to the medium, as appropriate.

After completion of the culture, 10 µl of an MTT labeling reagent was added to the culture, and the obtained mixture was further cultured for 4 hours. Subsequently, 100 µl of a lysate was added, and the reaction mixture was then incubated overnight. Thereafter, the absorbance at 550 nm was measured for each well, and the numerical values of blank wells were then subtracted from the obtained values. The experiment was independently carried out four times.

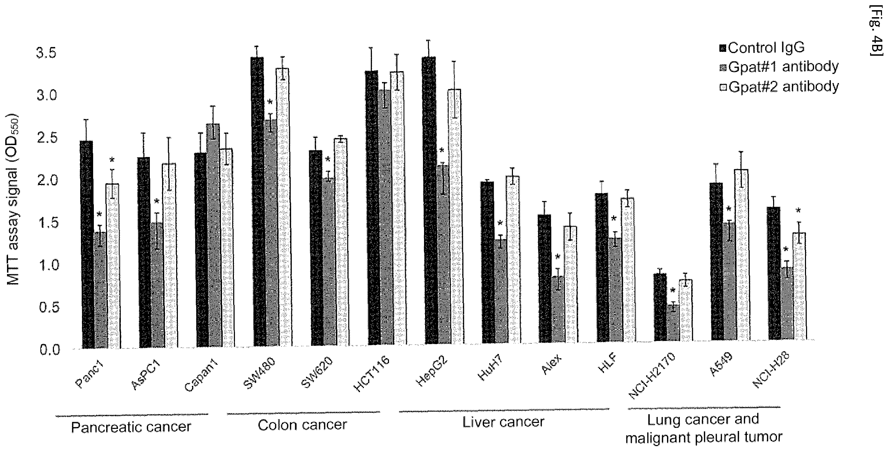

As a result, it was confirmed that the Gpat#1 antibody and the Gpat#2 antibody exhibit cell growth inhibitory effects on various human cancer cell lines, in comparison to the control antibody (Figs. 4A and 4B). The same assay was carried out using the antibodies having different concentrations. As a result, it was confirmed that these cell growth inhibitory effects depend on the antibody concentration (data are not shown herein).

### [Example 5: Neutralization of cell growth inhibitory effects by heparin]

Whether or not the cell growth inhibitory effects of the Gpat#1 antibody and the Gpat#2 antibody on the NUGC3 cell line are neutralized by heparin was studied.

The Gpat#1 antibody and the Gpat#2 antibody (20 µg/ml) were incubated with 60 µg/ml or 100 µg/ml low molecular weight heparin (H3149, Sigma Aldrich) at room temperature for 3 hours, and were then added to a well containing NUGC3 (6.0 x 10³ cells). Thereafter, the same MTT assay as that of Example 4 was carried out.

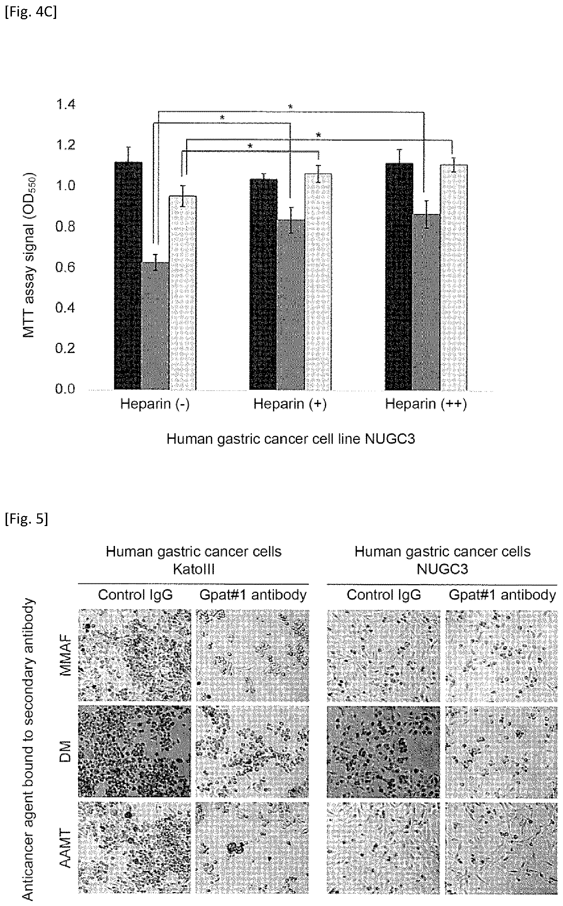

As a result, as shown in Fig. 4C, the cell growth inhibitory effects were reduced in a case where heparin was added (heparin (+) and heparin (++)) in comparison to a case where heparin was not added (heparin (-)). From these results, it was reconfirmed that the antigen recognized by these antibodies is sulfated glycosaminoglycan (heparin).

The same results were confirmed in other cell lines (data are not shown herein).

### [Example 6: Verification of effects of combined use with anticancer agent]

It was demonstrated that cell death is induced to human cancer cells by the combined use of the antibody of the present invention and a drug-conjugated secondary antibody.

As drugs, known anticancer agents, namely, monomethyl auristatin F (MMAF), duocarmycin (DM), and amanitin (AAMT) were selected, and anti-human IgG secondary antibodies (Moradec LLC), to which these anticancer agents had previously been bound, were used.

The human gastric cancer cell lines Katolll and NUGC3 were seeded in a 96-well plate at a cell concentration of 1.0 x 10⁴ cells, and were then cultured at 37°C for 4 hours. Subsequently, the Gpat#1 antibody (1.0 µg/well) or the same control IgG as that of Example 3 was added to each well, and 1 hour after the addition, the secondary antibody (0.5 µg/well) was added thereto, followed by further performing a culture for 3 days.

Three days later, the form of the cells was observed under a microscope. As a result, it was confirmed that inhibition of the growth of the cells and cell death were more clearly induced by the combined use of the Gpat#1 antibody and an anticancer agent, than in the case of the combined use of the control IgG and an anticancer agent (Fig. 5).

### [Example 7: Detection of anti-sulfated glycosaminoglycan antibody in serum]

Using the serum of a patient with diffuse-type gastric carcinoma and non-cancerous control serum (BioreclamationlVT), and also using Protein-G Magnetic Bead (BioRad, USA) in the same manner as that of Example 2, the presence of an anti-sulfated glycosaminoglycan antibody was detected in the serum.

Each serum (10 µl) was allowed to react with the Protein-G Magnetic Bead, and the obtained mixture was then blended with FITC-Conjugated Heparin (Life Technologies, USA), so that they were reacted with each other. The beads were washed, and were then observed under a fluorescence microscope. As a result, an aggregate of FITC-heparin generated as a result of the binding reaction with the antibody was observed in the serum of the patient with diffuse-type gastric carcinoma, whereas such an aggregate was not observed in the non-cancerous control serum (data are not shown herein). From these results, it was demonstrated that the anti-sulfated glycosaminoglycan antibody (anti-heparin antibody) is present not only in the cancer tissues but also in the serum, and thus, it was considered that the anti-sulfated glycosaminoglycan antibody would increase in cancer patients.

### [Example 8: Production of modified antibodies and cell growth inhibitory effects thereof]

With regard to the Gpat#1 antibody, based on the data of somatic hypermutation found at a high frequency in clones in gastric cancer samples, by using a known genetic recombination method and the protein expression system described in Example 1, modified antibodies (a modified Gpat#1 antibody 1 to a modified Gpat#1 antibody 5) each comprising an amino acid substitution in the heavy chain variable region were produced (Fig. 6A). All of these modified antibodies comprise a modification by substitution, and the position of the amino acid to be modified and the amino acid residue to be substituted were determined by extracting an amino acid sequence similar to the Gpat#1 antibody on the basis of immunoglobulin sequence data regarding the gastric cancer cases described in Example 1. The light chain of each of these modified antibodies has the same amino acid sequence as that of the Gpat#1 antibody, and the sequence of the light chain variable region is set forth in SEQ ID NO: 2.

Specifically, the modified Gpat#1 antibody 1 has the substitution of the amino acid at position 51 Asp with Asn in the amino acid sequence (SEQ ID NO: 1) of the heavy chain variable region of Gpat#1 antibody (SEQ ID NO: 27). This position corresponds to the position of the amino acid at position 4 in the amino acid sequence (SEQ ID NO: 14) of the CDR2 region of the Gpat#1 antibody.

The modified Gpat#1 antibody 2 has the substitution of the amino acid at position 54 Ser with Thr in SEQ ID NO: 1 (SEQ ID NO: 28). This position corresponds to the position of the amino acid at position 7 in the amino acid sequence (SEQ ID NO: 14) of the CDR2 region of the Gpat#1 antibody.

The modified Gpat#1 antibody 3 has the substitution of the amino acid at position 56 Ala with Gly in SEQ ID NO: 1 (SEQ ID NO: 29). This position corresponds to the position of the first amino acid in the framework (FR) 3 region adjacent to the CDR2 region of the Gpat#1 antibody.

The modified Gpat#1 antibody 4 has the substitution of the amino acid at position 66 Ile with Thr in SEQ ID NO: 1 (SEQ ID NO: 30). This position corresponds to the position of the amino acid at position 11 in the FR3 region of the Gpat#1 antibody.

The modified Gpat#1 antibody 5 has the substitution of the amino acid at position 102 Pro with Ser in SEQ ID NO: 1 (SEQ ID NO: 31). This position corresponds to the position of the amino acid at position 9 in the amino acid sequence (SEQ ID NO: 15) of the CDR3 region of the Gpat#1 antibody.

The MTT assay was performed on the obtained modified antibodies in the same manner as that of Example 4. As a result, all of the modified antibodies exhibited cell growth inhibitory effects that were equivalent to or greater than the effects of Gpat#1, and among others, the modified Gpat#1 antibody 1, the modified Gpat#1 antibody 3, and the modified Gpat#1 antibody 4 provided higher cell growth inhibitory effects than Gpat#1 (Fig. 6B).

### Industrial Applicability

According to the present invention, provided is a novel useful means for treating and detecting diseases such as cancer, by targeting sulfated glycosaminoglycan found on the cells of various cancers, such as diffuse-type gastric carcinoma as a typical example, for which there have conventionally been no effective therapeutic means.

All publications, patents and patent applications cited in the present description are incorporated herein by reference in their entirety.

## Claims

1. An antibody that binds to sulfated glycosaminoglycan and has cell growth inhibitory activity.

2. The antibody according to claim 1, wherein the sulfated glycosaminoglycan is heparan sulfate glycosaminoglycan or heparin.

3. The antibody according to claim 1, wherein the sulfated glycosaminoglycan is heparin.

4. The antibody according to any one of claims 1 to 3, which has cell growth inhibitory activity in the absence of an effector cell and a complement.

5. An antibody binding to sulfated glycosaminoglycan, which is any one selected from the following (1) to (10):
(1) an antibody having a heavy chain variable region comprising CDR1 consisting of the amino acid sequence set forth in SEQ ID NO: 13, CDR2 consisting of the amino acid sequence set forth in SEQ ID NO: 14, and CDR3 consisting of the amino acid sequence set forth in SEQ ID NO: 15;
(2) an antibody having a light chain variable region comprising CDR1 consisting of the amino acid sequence set forth in SEQ ID NO: 16, CDR2 consisting of the amino acid sequence set forth in SEQ ID NO: 17, and CDR3 consisting of the amino acid sequence set forth in SEQ ID NO: 18;
(3) an antibody having a heavy chain variable region comprising CDR1 consisting of the amino acid sequence set forth in SEQ ID NO: 19, CDR2 consisting of the amino acid sequence set forth in SEQ ID NO: 20, and CDR3 consisting of the amino acid sequence set forth in SEQ ID NO: 21;
(4) an antibody having a light chain variable region comprising CDR1 consisting of the amino acid sequence set forth in SEQ ID NO: 22, CDR2 consisting of the amino acid sequence set forth in SEQ ID NO: 23, and CDR3 consisting of the amino acid sequence set forth in SEQ ID NO: 24;
(5) an antibody having a heavy chain variable region having the amino acid sequence set forth in SEQ ID NO: 1 or 4, and a light chain variable region having the amino acid sequence set forth in SEQ ID NO: 2 or 6;
(6) an antibody having a heavy chain variable region having the amino acid sequence set forth in SEQ ID NO: 7 or 10, and a light chain variable region having the amino acid sequence set forth in SEQ ID NO: 8 or 12;
(7) an antibody having a heavy chain variable region consisting of an amino acid sequence having a sequence identity of 90% or more to SEQ ID NO: 1, and a light chain variable region consisting of an amino acid sequence having a sequence identity of 90% or more to SEQ ID NO: 2;
(8) an antibody having a heavy chain variable region consisting of an amino acid sequence having a sequence identity of 90% or more to SEQ ID NO: 7, and a light chain variable region consisting of an amino acid sequence having a sequence identity of 90% or more to SEQ ID NO: 8;
(9) an antibody having a heavy chain and/or a light chain consisting of an amino acid sequence comprising a substitution, deletion, and/or addition of one or several amino acids in the heavy chain variable regions and/or the light chain variable regions of the antibodies (1) to (6); and
(10) the antibody (9) having one or more substitutions selected from the substitution of the amino acid at position 51 Asp with Asn, the substitution of the amino acid at position 56 Ala with Gly, and the substitution of the amino acid at position 66 Ile with Thr, in the amino acid sequence set forth in SEQ ID NO: 1.

6. A cell growth inhibitor comprising the antibody according to any one of claims 1 to 5 as an active ingredient.

7. The cell growth inhibitor according to claim 6, which is used in combination with a drug having cytotoxicity.

8. The cell growth inhibitor according to claim 6 or 7, which is used to inhibit the growth of cancer cells.

9. The cell growth inhibitor according to claim 8, wherein the cancer cells are cancer cells in gastric cancer, pancreatic cancer, colon cancer, liver cancer, lung cancer, and/or malignant pleural tumor.

10. A targeting reagent for drug delivery, comprising the antibody according to any one of claims 1 to 5.

11. A pharmaceutical composition comprising, as an active ingredient, the cell growth inhibitor according to any one of claims 6 to 9, or the targeting reagent according to claim 10.

12. An agent for detecting cancer, comprising the antibody according to any one of claims 1 to 5.

13. The detecting agent according to claim 12, for use in detecting cancer selected from gastric cancer, pancreatic cancer, colon cancer, liver cancer, lung cancer, and malignant pleural tumor.

14. A method for treating cancer in a subject, comprising administering the cell growth inhibitor according to any one of claims 6 to 9, the targeting reagent according to claim 10, or the pharmaceutical composition according to claim 11, to the subject.
